(19) Europäisches Patentamt
European Patent Office
Office européen des brevets

(11) Publication number: 0 337 136 A2

# (12) EUROPEAN PATENT APPLICATION

(21) Application number: 89104548.6

(51) Int. Cl.⁴: A61K 31/445

(22) Date of filing: 15.03.89

The title of the invention has been amended (Guidelines for Examination in the EPO, A-III, 7.3).

(30) Priority: 17.03.88 US 169269

(43) Date of publication of application: 18.10.89 Bulletin 89/42

(84) Designated Contracting States: AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Palfreyman, Michael G.
11515 Applejack Court
Cincinnati Ohio 45249(US)
Inventor: Carr, Albert A.
6693 East Farm Acres Drive
Cincinnati Ohio 45237(US)

(74) Representative: Vossius & Partner
Siebertstrasse 4 P.O. Box 86 07 67
D-8000 München 86(DE)

## (54) Piperidine derivatives in the treatment of extrapyramidal side effects associated with neuroleptic therapy.

(57) The present invention pertains to the use of a class of serotonin $5HT_2$ antagonists for the preparation of pharmaceutical compositions for the treatment of the extrapyramidal side effects associated with neuroleptic drug therapy.

EP 0 337 136 A2

## PHARMACEUTICAL COMPOSITION FOR THE TREATMENT OF THE EXTRAPYRAMIDAL SIDE EFFECTS ASSOCIATED WITH NEUROLEPTIC THERAPY

The present invention pertains to a method for the preparation of pharmaceutical compositions for the treatment of the extrapyramidal side effects associated with neuroleptic drug therapy.

The administration of neuroleptic agents such as chlorpromazine, haloperidol, fluphenazine, trifluoperazine, etc., typically produce extrapyramidal symptoms in a significant percentage of the patients treated with these agents. These extrapyramidal side effects (EPS) can manifest themselves in a variety of ways. Some patients experience a parkinsonian-like syndrome, wherein they experience muscular rigidity and tremors. Others experience akathisia, which can be characterized as a compelling need for the patient to be in constant movement. A few patients experience acute dystonic reactions, such as facial grimacing and torticollis.

Typically these side effects are controlled with anticholinergic agents such as benztropine or trihexyphenidyl. Neuroleptic agents are dopaminergic antagonists. Thus, it is believed that the anticholinergic agents relieve EPS by correcting the imbalance of cholinergic and dopaminergic neuronal responses occuring in the corpus striatum of these patients.

Recent evidence suggests that there may be alternative means by which these extrapyramidal side effects may be controlled. It has been hypothesized by various authors that serotonergic receptors may also be involved with these extrapyramidal side effects. They base their hypothesis on the fact that the serotonin neurotransmitter (5HT) is also extensively involved in controlling locomotory function in the corpus striatum.

In accordance with the present invention it has been discovered that the following class of serotonin $5HT_2$ antagonists are useful for the preparation of a pharmaceutical composition for treating extrapyramidal side effects.

They contain compounds of the formula:

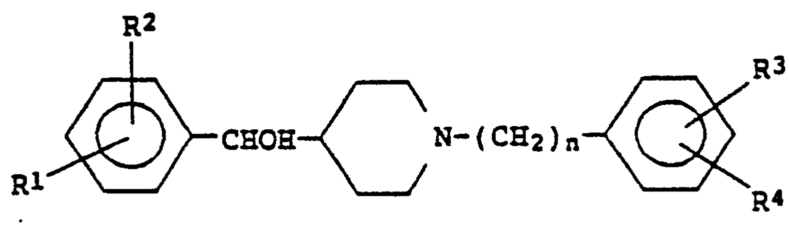

Formula I

wherein:
each of $R^1$, $R^2$, $R^3$, and $R^4$ are independently selected from the group consisting of hydrogen, a $C_{1-6}$ alkyl group, halogen, trifluoromethyl, hydroxy, a $C_{1-6}$ alkoxy group, or an amino group; n is 2, 3, or 4; and the pharmaceutically acceptable acid addition salts thereof;

As used in this application:

a) The term $C_{1-6}$ alkyl refers to a straight chain or branched alkyl group containing up to 6 carbon atoms. Representative examples of suitable alkyl groups include, methyl, ethyl, propyl, isopropyl, n-butyl, t-butyl, pentyl, hexyl, cyclopropyl, and cyclopentyl. Methyl and ethyl are currently preferred.

b) The term halogen refers to a fluorine, bromine, chlorine or iodine atom. Fluorine and chlorine are currently preferred.

c) The term $C_{1-6}$ alkoxy refers to a straight chain or branched alkoxy group containing up to 6 carbon atoms. Representative examples of suitable alkoxy groups include methoxy, ethoxy, propoxy, isopropoxy, butoxy, pentoxy, and hexyloxy.

d) The term hydroxy in this application refers to the following substituent -OH.

e) The term amino refers to $-NH_2$.

f) The term "patient" as used herein is taken to mean warm-blooded animals, such as mammals, for example, dogs, rats, mice, cats, guinea pigs, and primates, including humans.

g) The phrase "extrapyramidal side effects" or the abbreviation "EPS" refers to the parkinsonian-like syndrome, akasthisia, and dystonic reactions that are typically associated with the administration of neuroleptic agents.

h) The terms, "treat or treatment", refers to the ability of these agents to either stop, lessen the severity of, or prevent the occurrence of the patient's extrapyramidal side effects.

The expression "pharmaceutically acceptable acid addition salts" is intended to apply to any non-toxic organic or inorganic acid addition salt of the base compounds represented by Formula I. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic and sulfonic acids such as methane sulfonic acid and 2-hydroxyethane sulfonic acid.

The compounds represented by Formula I exist as optical isomers. Any reference in this application to the compounds of Formula I, is meant to encompass a specific isomer or a mixture of isomers. Evidence has been recently discovered that suggests that the levo(-) isomer may be a more potent $5HT_2$ antagonist than the dextro(+) isomer. Thus, it may be desirable to prepare pharmaceutical compositions containing the levo isomer for the treatment of this condition.

In those instances where $R^1$-$R^4$ are other than hydrogen, the substituents may be located at any position of the phenyl ring (i.e., meta, para, or ortho). Para is currently preferred for monosubstituted phenyl moieties. The 2,3-, 2,4- 2,5-, 3,4-, or 3,5-disubstitued phenyl moieties are also embraced herein. $R^1$, $R^2$, $R^3$, and $R^4$ can each be the same or different substituents.

It is currently preferred for n to be either 2 or 3, with 2 being most preferred. It is also currently preferred for $R^3$ and $R^4$ to be hydrogen.

Representative examples of preferred compounds include:

1) α-phenyl-1-(2-phenethyl)-4-piperidine methanol;
2) α-phenyl-1-(3-phenylpropyl)-4-piperidine methanol;
3) α-(4-methylphenyl)-1-(2-phenethyl)-4-piperidine methanol;
4) α-(4-methoxyphenyl)-1-(2-phenethyl)-4-piperidine methanol;
5) α-(3,5-dimethylphenyl)-1-(2-phenethyl)-4-piperidine methanol;
6) α-(3-(trifluoromethyl)phenyl)-1-(2-phenethyl)-4-piperidine methanol;
7) α-(2,3-dimethoxyphenyl)-1-(2-phenethyl)-4-piperidine methanol;
8) α-(4-fluorophenyl)-[1-(2-phenylethyl)-4-piperidinyl]-methanol;
9) α-phenyl-[1-(4-phenylbutyl)-4-piperidinyl]-methanol;
10) α-(3,4-dimethoxyphenyl-[1-(2-phenylethyl)-4-piperidinyl]-methanol;
11) α-phenyl-[1-(4-aminophenylethyl)-4-piperidinyl]-methanol;
12) α-phenyl[1-(4-methoxyphenylethyl)-4-piperidinyl]-methanol;
13) α-(4-methoxyphenyl)-[1-(4-methoxy-phenylethyl)-4-piperidinyl]-methanol;
14) α-(2,3-dimethoxyphenyl)-[1-(4-methoxyphenylethyl)-4-piperidinyl-methanol;
15) α-phenyl-[1-(4-methoxyphenylethyl)-4-piperidinyl)-methanol;
16) α-phenyl-[1-(4-fluorophenylethyl)-4-piperidinyl]-methanol;
17) α-(4-hydroxyphenyl)-[1-(2-phenylethyl)-4-piperidinyl]-methanol;
18) α-(3,4-dihydroxyphenyl)-[1-(2-phenylethyl)-4-piperidinyl]-methanol, and;
19) α-(3,4-dichlorophenyl)-1-(2-phenylethyl)-4-piperidine methanol.

The compounds of Formula I, their methods of preparation and their use as serotonin $5HT_2$ antagonists are known in the art. European Patent Application 0 208 235 discloses these compounds and several methods for preparing these compounds. Any of these methods, or any other method known in the art, are suitable for preparing the compounds to be utilized in the method of the present invention.

The compounds of Formula I are useful for the preparation of pharmaceutical compositions for treating, that is for stopping, lessening the severity of, or preventing the occurence of, the parkinsonian-like syndrome, akathisia, and dystonic reactions that are commonly associated with neuroleptic drug therapy. These pharmaceutical compositions can be administered to terminate or reduce the severity of an acute extrapyramidal reaction, or they can be administered prophylactically in order to prevent the occurrence of extrapyramidal side effects.

The amount of compound in the pharmaceutical compositions required to produce these effects (an anti-EPS amount) will vary with the patient, the particular compound chosen, the particular neuroleptic being concurrently administered, the quantity of neuroleptic being concurrently administered, the presence of other underlying disease states in the patient, the severity of the patient's side effects, whether an acute EPS attack is being terminated or whether the pharmaceutical compositions are being administered

prophylactically to prevent the occurrence of EPS, as well as other medications which are being concurrently administered to the patient. Generally though, a patient's side effects will respond to a dosage range of from 0.02-10mg/kg/day of the active ingredient.

The pharmaceutical compositions containing the compounds of Formula I can be administered either orally or parenterally (i.e. intravenously, intramuscularly, subcutaneously, etc.). Repetitive daily administration may be desirable and will vary with the dosage form utilized as well as the other parameters described above for the quantity of compound required.

The pharmaceutical compositions containing the compounds of Formula I can have a variety of dosage forms, such as for example, tablets, capsules, solutions, elixirs, sterile solutions for injection and sustained release preparations. Methods for producing these dosage forms are well known in the art and are disclosed in European Patent Application 0 208 235.

## Claims

1.Use of a compound of the formula:

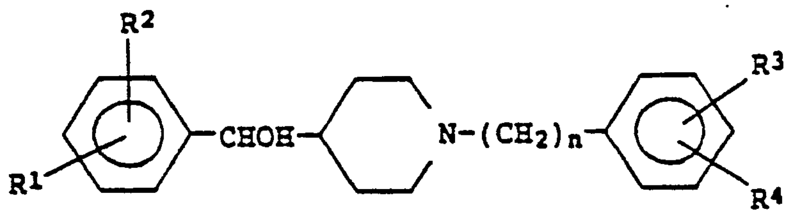

wherein:
each of $R^1$, $R^2$, $R^3$, and $R^4$ are independently selected from the group consisting of hydrogen, a $C_{1-6}$ alkyl group, halogen, trifluoromethyl, hydroxy, a $C_{1-6}$ alkoxy group, or an amino group; n is 2, 3, or 4; and the pharmaceutically acceptable acid addition salts thereof for the preparation of a pharmaceutical composition for the treatment of the extrapyramidal side effects associated with neuroleptic drug therapy.

2.Use according to claim 1, wherein said compoung is a α-phenyl-1-(2-phenethyl)-4-piperidine methanol.

3.Use according to claim 1, wherein said compoung is α-phenyl-1-(3-phenpropyl)-4-piperidine methanol.

4.Use according to claim 1, wherein said compoung is α-(4-methylphenyl)-1-(2-phenethyl)-4-piperidine methanol.

5.Use according to claim 1, wherein said compoung is α-(4-methoxyphenyl)-1-(2-phenethyl)-4-piperidine methanol.

6.Use according to claim 1, wherein said compound is α-(3,5-dimethylphenyl)-1-(2-phenethyl)-4-piperidine methanol.

7.Use according to claim 1, wherein said compound is α-(3-(trifluoromethyl)phenyl)-1-(2-phenethyl)-4-piperidine methanol.

8.Use according to claim 1, wherein said compound is α-(2,3-dimethoxylphenyl)-1-(2-phenethyl)-4-piperidine methanol.

9.Use according to claim 1, wherein said compound is α-(4-fluorophenyl)-[1-(2-phenylethyl)-4-piperidinyl]-methanol.

10.Use according to claim 1, wherein said compound is α-phenyl-[1-(4-phenylbutyl)-4-piperidinyl]-methanol.

11.Use according to claim 1, wherein said compound is α-(3,4-dimethoxyphenyl)-[1-(2-phenylethyl)-4-piperidinyl]-methanol.

12.Use according to claim 1, wherein said compound is α-phenyl-[1-(4-aminophenylethyl)-4-piperidinyl]-methanol.

13.Use according to claim 1, wherein said compound is α-phenyl[1-(4-methoxyphenylethyl)-4-piperidinyl]-methanol.

14.Use according to claim 1, wherein said compound is α-(4-methoxyphenyl)-[1-(4-methoxyphenylethyl)-4-piperidinyl]-methanol.

15.Use according to claim 1, wherein said compound is α-(2,3-dimethoxyphenyl)-[1-(4-methoxyphenylethyl)-4-piperidinyl]-methanol.

16.Use according to claim 1, wherein said compound is α-phenyl-[1-(4-methoxyphenylethyl)-4-piperidinyl]-methanol.

17.Use according to claim 1, wherein said compound is α-phenyl-[1-(4-fluorophenylethyl)-4-piperidinyl]-methanol.

18.Use according to claim 1, wherein said compound is α-(4-hydroxyphenyl)-[1-(2-phenylethyl)-4-piperidinyl]-methanol.

19.Use according to claim 1, wherein said compound is α-(3,4-dihydroxyphenyl)-[1-(2-phenylethyl)-4-piperidinyl]-methanol.

20.Use according to claim 1 wherein said compound is α-(3,4-dichlorophenyl)-1-(2-phenylethyl)-4-piperidine methanol.